# EUROPEAN PATENT SPECIFICATION

(11) **EP 2 592 928 B1**
(45) Date of publication and mention of the grant of the patent: **30.04.2014**
(21) Application number: 11738422.2
(22) Date of filing: 11.07.2011
(51) Int. Cl.: A01N 1/00, A61K 8/41, A61K 8/42, A61K 8/44, A61Q 5/02, A61Q 17/00, A61Q 19/10, A01N 33/12, A01N 25/30

(54) **PRESERVATIVE SYSTEM AND COMPOSITION BASED ON GLYCINATE AND DIHYDROXYPROPYL QUATERNARY AMMONIUM SALT COMBINATION**
KONSERVIERUNGSSYSTEM UND ZUBEREITUNG BASIEREND AUF EINER KOMBINATION AUS GLYCINAT UND EINEM DIHYDROXY-PROPYL AMMONIUM SALZ
SYSTÈME DE CONSERVATEUR ET COMPOSITION À BASE D'UNE COMBINAISON DE SEL DE GLYCINATE ET DE SEL D'AMMONIUM QUATERNAIRE DE DIHYDROXYPROPYLE

(30) Priority: 12.07.2010 US 834131
(43) Date of publication of application: 22.05.2013
(73) Proprietor: Unilever PLC, London Greater London EC4Y 0DY (GB); Unilever NV, 3013 AL Rotterdam (NL)
(72) Inventor: MITCHELL, Deidre, Lee, CT 06611 (US); BETKOSKI, Roberta, Marie, CT 06611 (US); ANANTHAPADMANABHAN, Kavssery, Parameswaran, CT 06611 (US); YANG, Lin, CT 06611 (US)
(74) Representative: Tansley, Sally Elizabeth
(86) International application number: PCT/EP2011/061728
(87) International publication number: WO 2012/007412

(56) References cited:
- WO-A1-2006/026875
- WO-A2-2009/071776
- DATABASE CA [Online] CHEMICAL ABSTRACTS SERVICE, COLUMBUS, OHIO, US; YOSHIMURA, TOSHITSUGU ET AL: "Quaternary ammonium compounds as bactericides and their preparation", XP002665509, retrieved from STN Database accession no. 112:157670 & JP 1 233264 A (TAMURA SEIYAKU K. K., JAPAN) 19 September 1989 (1989-09-19)
- DATABASE CA [Online] CHEMICAL ABSTRACTS SERVICE, COLUMBUS, OHIO, US; MAMADA, HIROSHI ET AL: "Antimicrobial characteristics and adsorption to halogenated glycerine", XP002665510, retrieved from STN Database accession no. 112:73657 & MAMADA, HIROSHI ET AL: "Antimicrobial characteristics and adsorption to halogenated glycerine", BOKIN BOBAI , 17(9), 413-18 CODEN: BOBODP; ISSN: 0385-5201, 1989,

## Description

### BACKGROUND OF THE INVENTION

### Field of the Invention

The invention concerns an anti-bacterial active mixture and use in personal care products.

### The Related Art

Water containing formulations are prone to experience bacterial contamination. Preservatives are vitally necessary in defending against micro organisms. Gram negative bacilli such as *Pseudomonas aeruginosa* are particularly nasty organisms. Indeed, the U.S. Federal Drug Administration classifies *Pseudomonas aeruginosa* as an "objectionable organism". Medically this bacteria is a significant human pathogen.

Formulations intended for pharmaceutical and personal care compositions are highly affected by the problem.

Part of the issue involves ability of the micro organism to mutate. New preservatives must continually be developed to fight the contamination. Consequently, there is a need for new preservative systems that can meet the challenge of gram negative bacteria, and most especially of *Pseudomonas aeruginosa.*

WO 96/35410 (Henkel) reports use of dihydroxypropyl trimethyl ammonium chloride salts formulated into shampoos. These compositions further contain zwitterionic surfactants such as cocoalkyl dimethyl ammonium glycinate. These zwitterionic glycinates are different from the anionic glycinates of the present invention.

WO-A-2009/071776 describes an antimicrobial composition comprising a combination of a lipoamino acid preferably comprising an alkylated C₈₋₁₈ carbon atom chain, a hydroxylated C₈₋₁₈ fatty acid and an alkyl ether of glycerol.

WO-A-2006/026875 describes acid compounds of general formula R²XY(CH₂)COOH, wherein Y is selected from O and NH, X is selected from CO and CH₂, and R² is a branched saturated or unsaturated C₇₋₁₅ hydrocarbon moiety, which acids are said to be useful as antibacterial or antifungal compounds in consumer products.

JP-A-19880061389 describes quaternary ammonium salts selected from monoammonium salts of general formula [R₁R₂N(CH₃)CH₂CH(OH)CH₂(OH]⁺X⁻ and [R₂N(CH₃)₂CH₂CH(OH)CH₂N(CH₃)₂R₂]²⁺2X⁻, where R₁ and R₂ are C₈₋₁₈ alkyl.

"Antimicrobial characteristics and absorption to halogenated glycerine", Bokin Bobai, 17(9), 413-18, 1989 describes a preservative system and composition based on glycinate and dihydroxypropyl quarternary ammonium salt combination.

### SUMMARY OF THE INVENTION

A preservative system is provided which includes:
(i) sodium cocoyl glycinate; and
(ii) 2,3-dihydroxypropyl trimethylammonium chloride; and
wherein the system exhibits a Log Reduction against *Pseudomonas aeruginosa* of at least 2 within 48 hours of application to an aqueous system.

Further provided is a personal care composition which includes:
(i) from 1 to 20% by weight of sodium cocoyl glycinate;
(ii) from 0.05 to 10% by weight of 2,3-dihydroxypropyl trimethyl ammonium chloride; and
(iii) water

### DETAILED DESCRIPTION OF THE INVENTION

Now it has been found that gram negative bacteria, especially *Pseudomonas aeruginosa,* can be killed by a combination of sodium cocoyl glycinate and 2,3-dihydroxypropyl trimethyl ammonium chloride. The combination in a personal care composition can reduce the amount of *Pseudomonas aeruginosa* by a Log Reduction of at least 2, preferably at least 3.5 and optimally at least 5 within 48 hours of application to an aqueous system.

For the preservative system the ratio by weight of the sodium cocoyl glycinate and 2,3-dihydroxypropyl trimethyl ammonium chloride salts may respectively range from 200:1 to 1:5, preferably from 150:1 to 1:1, optimally from 100:1 to 1:1.

In personal care compositions the amounts of the glycinate salt may range from 1 to 20%, preferably from 3 to 15%, and optimally from 3 to 8% by weight of the composition.

A second element of the present invention is that of a 2,3-dihydroxypropyl trimethyl ammonium chloride salt.

These salts may be obtained in a variety of synthetic procedures, most particularly by hydrolysis of chlorohydroxypropyltri(C₁ alkyl or hydroxyalkyl) ammonium salts. Ordinarily the C₁ alkyl or hydroxyalkyl constituent on the quatemized ammonium group will be methyl, hydroxymethyl and mixtures thereof.

In personal care compositions the amounts of the 2,3-dihydroxypropyl trimethyl ammonium chloride salts may range from 0.05 to 10%, preferably from 0.1 to 5%, more preferably from 0.3 to 30%, optimally from 0.5 to 1% by weight of the composition.

Advantageously the pH of compositions of this invention may range from pH 5.5 to 8, preferably from 6 to 7.8, and optimally from 6.8 to 7.8.

Personal care compositions containing the preservative system of this invention ordinarily will also contain water. These compositions may include a hydrophobic phase thereby forming an emulsion. Water-in-oil and oil-in-water as well as triplex emulsions may be useful as carriers according to the present invention. Amounts of water may range from 10% to 99%, preferably from 30 to 90%, and optimally from 50 to 70% water by weight of the composition.

Personal care compositions are products that include, but are not limited to, shampoos, bodywash, liquid and bar type hand cleansers, toothpastes, face and body lotions and skin creams. Common to most of these products are surfactant systems. Indeed, surfactant systems that foam (i.e. cleansing formulations) are particularly prone to microbial contamination because of high water content in these formulations. Preservative systems of this invention are therefore especially useful for cleansing formulations.

Consequently, compositions of this invention may include a surfactant. Amounts of the surfactant may range from 0.1 to 30%, preferably from 1 to 20%, and optimally from 1 to 15% by weight of the personal care composition.

The surfactant may be selected from the group consisting of anionic, nonionic, cationic and amphoteric actives. Particularly preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) and trialkylamine oxides are also suitable nonionic surfactants.

Preferred anionic surfactants include salts of the following: fatty acids (i.e. soap), alkyl ether sulfates and sulfonates, alkyl sulfates and sulfonates, alkylbenzene sulfonates, alkyl and dialkyl sulfosuccinates, C₈-C₂₀ acyl isethionates, C₈-C₂₀ alkyl ether phosphates, C₈-C₂₀ sarcosinates, C₈-C₂₀ acyl lactylates, sulfoacetates and combinations thereof. Most preferred is sodium cocoyl isethionate.

Useful amphoteric surfactants include cocoamidopropyl betaine, C₁₂-C₂₀ trialkyl betaines, sodium lauroamphoacetate, and sodium laurodiamphoacetate.

Humectants may be present in the personal care compositions. These are generally polyhydric alcohol-type materials. Typical polyhydric alcohols include glycerin, propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of humectant may range anywhere from 0.2 to 40%, preferably between 1 and 25%, most preferably between 2 and 15% by weight of the composition. Most preferred is glycerin as an humectant or moisturizer.

Emollient materials may be formulated into the compositions. These may be natural or synthetic esters and hydrocarbons. Amounts of the emollients may range anywhere from 0.1 to 20%, preferably between 1 and 10% by weight of the composition.

Among the ester emollients are:
(a) Alkyl esters of saturated fatty acids having 10 to 24 carbon atoms. Examples thereof include behenyl neopentanoate, isononyl isonanonoate, isopropyl myristate and octyl stearate.
(b) Ether-esters such as fatty acid esters of ethoxylated saturated fatty alcohols.
(c) Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of C₁-C₃₀ alcohols.
(d) Wax esters such as beeswax, spermaceti wax and tribehenin wax.
(e) Sugar ester of fatty acids such as sucrose polybehenate and sucrose polycottonseedate.

Natural ester emollients principally are based upon mono-, di- and triglycerides. Representative glycerides include cottonseed oil, borage oil, borage seed oil, primrose oil, castor and hydrogenated castor oils, rice bran oil, soybean oil, olive oil, safflower oil, shea butter, jojoba oil and combinations thereof. Animal derived emollients are represented by lanolin oil and lanolin derivatives. Amounts of the natural esters may range from 0.1 to 20% by weight of the compositions.

Hydrocarbons suitable for the compositions include petrolatum, mineral oil, C₁₁-C₁₃ isoparaffins, polybutenes, and especially isohexadecane, available commercially as Permethyl 101A from Presperse Inc.

Fatty acids having from 10 to 30 carbon atoms may also be suitable as components. Illustrative of this category are pelargonic, lauric, myristic, palmitic, stearic, isostearic, oleic, linoleic, linolenic and behenic acids.

Sunscreen agents may also be included in compositions of the present invention. Particularly preferred are such materials as ethylhexyl p-methoxycinnamate, available as Parsol MCX®, Avobenzene available as Parsol 1789®, and benzophenone-3 also known as Oxybenzone. Inorganic sunscreen actives may be employed such as microfine (1 to 100 nm) titanium dioxide and zinc oxide. Amounts of the sunscreen agents when present may generally range from 0.1 to 30%, preferably from 2 to 20%, optimally from 4 to 10% by weight of the composition.

Structurants for aqueous compositions may be selected from inorganic water structurants, charged polymeric water structurants, water soluble polymeric structurants, associative water structurants, and mixtures thereof. Non-limiting examples of inorganic water structurants include silicas, polymeric gellants such as polyacrylates, polyacrylamides, starches, modified starches, crosslinked polymeric gellants, copolymers, and mixtures thereof. Non-limiting examples of charged polymeric water structurants include Acrylates/Vinyl Isodecanoate Crosspolymer, Acrylates/C10-30 Alkyl Acrylate Crosspolymer, Carbomers, Ammonium Acryloyldimethyltaurate/VP Copolymer, Ammonium Acryloyidimethyltaurate/Beheneth-25 Methacrylate Crosspolymer, Acrylates/ Ceteth-20 Itaconate Copolymer, Polyacrylamide, and mixtures thereof. Non-limiting examples of water soluble polymeric structurants include cellulose gums and starches. Non-limiting examples of associative water structurants include xanthum gum, gellum gum, pectins, alginates such as propylene glycol alginate, and mixtures thereof.

Cationic deposition polymers may also be utilized. Non-limiting examples include polysaccharide polymers, such as cationic cellulose derivatives. Preferred are the salts of hydroxyethyl cellulose reacted with trimethyl ammonium substituted epoxide, referred to the industry as polyquaternium T10 which are available from Amerchol Corp. in their Polymer KG, JR and LR series of polymers.

Toothpastes formulated according to the present invention will generally contain a fluoride source to prevent dental caries. Typical anti-caries actives include sodium fluoride, stannous fluoride and sodium monofluoro phosphate. Amounts of these materials will be determined by the amount of fluoride releasable which should range between about 500 to about 1800 ppm of the composition. Other components of dentifrices can include desensitizing agents such as potassium nitrate and strontium nitrate, sweeteners such as sodium saccharine, aspartame, sucralose, and potassium acesulfam. Thickeners, opacifying agents, abrasives and colorants will normally also be present.

Colorants, opacifiers and abrasives may also be included in compositions of the present invention. Each of these substances may range from 0.05 to 5%, preferably between 0.1 and 3% by weight of the composition.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

The following examples will more fully illustrate the embodiments of this invention. All parts, percentages and proportions referred to herein and in the appended claims are by weight unless otherwise illustrated.

### EXAMPLE 1

Experiments were conducted to evaluate antimicrobial activity against *Pseudomonas aeruginosa* of aqueous formulas containing 2,3-dihydroxypropyl trimethyl ammonium chloride (Glyceryl Quat), sodium cocoyl glycinate and combinations of these two materials. Samples were prepared at room temperature by mixing together sodium cocoyl glycinate and/or Glyceryl Quat and water. The pH was adjusted by addition of aqueous hydrochloric acid to reach a 7.6 value. Amounts of each of the active components was 5%.

### Test Method: Challenge Test for the Evaluation of Preservative Capacity of Liquid Detergent and Personal Products

**Test Microorganism:** *Pseudomonas aeruginosa* ATCC 9027 obtained from American Type Culture Collection, Manassas, VA 20108.

**Procedure:** 20 grams of product were inoculated with 0.2 ml of the microorganism at a level of 1.06E+07 or Log 7. A single inoculation was performed. At 2 day, 7 day, 14 day and 21 day time points after inoculation a 1 gram sample was aseptically removed into a neutralizing fluid (TAT Broth) and plated using Letheen Agar. The plates were incubated at 30°C for 7 days. At the end of the incubation time the plates were removed and examined for microbial growth.

**Calculation of Log Reduction:** The microorganisms recovered were converted into Log₁₀ and compared to initial inoculum count which then determined the Log Reduction. Logarithmic functions are the inverse of exponential functions. Log reduction was determined by the initial inoculum count minus the counts at each sampling time point.

**TABLE I**

| | Log Reduction Value | | | |
|---|---|---|---|---|
| Component | 2 day | 7 day | 14 day | 21 day |
| Sodium Cocoyl | 1.1 | 1.1 | 0.8 | 0.2 |
| Glycinate | | | | |
| Glyceryl Quat | 2.1 | 1.5 | 2.8 | 3.4 |
| Sodium Cocoyl | | | | |
| Glycinate and | 6.0 | 6.0 | 6.0 | 6.0 |
| Glyceryl Quat (1:1) | | | | |

Based on the results recorded in the Table, it is seen that salt combinations of glycinate and dihydroxypropyl trimethyl ammonium salt (Glyceryl Quat) provide a strong immediate kill within 48 hours and continue effective throughout a 21 day period. The Log Reduction value for the 2 day application was 6.0. This value is known to be effective for commercial personal care compositions.

### EXAMPLES 2-4

A series of shower gel formulas suitable for the present invention are recorded in Table II, III and IV below.

**TABLE II**

| Ingredients | Example (wt %) | | |
|---|---|---|---|
| | **2** | **3** | **4** |
| Ammonium Laureth-3 Sulfate | 3.0 | 3.0 | 3.0 |
| Sodium Lauroamphoacetate | 16.7 | 16.7 | 16.7 |
| Sodium Cocoyl Glycinate | 10.0 | 8.0 | 6.5 |
| Lauric Acid | 0.9 | 0.9 | 0.9 |
| Trihydroxystearin | 2.0 | 2.0 | 2.0 |
| Guar Hydroxypropyltrimonium Chloride | 0.17 | 0.75 | 0.75 |
| Polyquaterium 10 | 0.45 | -- | -- |
| Polymethacrylamidopropyltrimonium Chloride | -- | 0.24 | -- |
| Polyquaternium-39 | -- | 0.81 | -- |
| PEG 90 M | 0.25 | -- | -- |
| PEG-14M | 0.45 | 2.45 | 2.45 |
| Linoleamidopropyl PG-Dimonium Chloride | -- | 1.0 | 4.0 |
| Glycerin | 1.4 | 4.9 | 4.9 |
| Sodium Chloride | 0.3 | 0.3 | 0.3 |
| Sodium Benzoate | 0.25 | 0.25 | 0.25 |
| Disodium EDTA | 0.13 | 0.13 | 0.13 |
| 2,3-Dihydroxypropyl Trimethylammonium Chloride | 1.0 | 2.0 | 0.3 |
| Glydant DMDM Hydantoin® | 0.37 | 0.37 | 0.37 |
| Citric Acid | 1.6 | 0.95 | 0.95 |
| Water | Balance | Balance | Balance |

A shampoo composition useful in the context of the present invention is described in Table III below.

**TABLE III**

| Ingredient | Weight % |
|---|---|
| Ammonium Laureth Sulfate | 12.00 |
| Ammonium Lauryl Sulfate | 2.00 |
| Cocoamidopropyl Betaine | 2.00 |
| Sodium Cocoyl Glycinate | 5.00 |
| 2,3-Dihydroxypropyl Trimethylammonium Chloride | 0.50 |
| Glycerin | 5.00 |
| Dihydroxypropyltrimonium Chloride | 5.50 |
| Ethylene Glycol Distearate | 1.50 |
| Cocomonoethanolamide | 0.80 |
| Cetyl Alcohol | 0.60 |
| Polyquatemium-10 | 0.50 |
| Dimethicone | 1.00 |
| Sodium Citrate | 0.40 |
| Citric Acid | 0.39 |
| Sodium Xylene Sulfonate | 1.00 |
| Fragrance | 0.40 |
| Sodium Benzoate | 0.25 |
| Kathon CG® | 0.0008 |
| Benzyl Alcohol | 0.0225 |
| Water | Balance |

An aerosol packaged foaming cleanser useful in the context of the present invention is described in Table IV.

**TABLE IV**

| Ingredient | Weight % |
|---|---|
| Sodium Cocoyl Glycinate | 10.00 |
| Glycerin | 10.00 |
| Maleated Soybean Oil | 5.00 |
| Silicone Urethane | 1.00 |
| Polyglycero-4 Oleate | 1.00 |
| Sodium C14-16 Olefin Sulfonate | 15.00 |
| Sodium Lauryl Ether Sulphate (25% Active) | 15.00 |
| Cocoamidopropylbetaine | 15.00 |
| DC 1784® (Silicone Emulsion 50%) | 5.00 |
| Polyquaternium-11 | 1.00 |
| Dihydroxypropyltrimonium Chloride | 1.00 |
| Water | Balance |

The foregoing description illustrates selected embodiments of the present invention. In light thereof, variations and modifications will be suggested to one skilled in the art.

## Claims

1. A preservative system comprising:
(I) sodium cocoyl glycinate; and
(II) 2,3-dihydroxypropyl trimethylammonium chloride; and
wherein the system exhibits a Log Reduction against Pseudomonas aeruginosa of at least 2 within 48 hours of application to an aqueous system.

2. The system according to claim 1 having a Log Reduction of at least 3.5 within 48 hours of application to an aqueous system.

3. A personal care composition comprising:
(i) from 1 to 20% by weight of a sodium ocoyl glycinate;
(ii) from 0.05 to 10% by weight of 2,3 -dihydroxypropyl trimethyl ammonium chloride; and
(iii) water.

4. The composition according to claim 3 further comprising from 0.1 to 30% of a surfactant.

5. The composition according to claim 4 wherein the surfactant is sodium cocoyl isethionate.

## Patentansprüche

1. Konservierungssystem, enthaltend
(i) Natriumcocoylglycinat und
(ii) 2,3-Dihydroxypropyltrimethylammoniumchlorid; und
wobei das System eine logarithmische Reduktion gegenüber Pseudomonas aeruginosa innerhalb von 48 Stunden der Anwendung bei einem wässrigen System von mindestens 2 zeigt.

2. System nach Anspruch 1,
das eine logarithmische Reduktion innerhalb von 48 Stunden der Anwendung bei einem wässrigen System von mindestens 3,5 zeigt.

3. Körperpflegezusammensetzung, die Folgendes aufweist:
(i) 1 bis 20 Gew.-% eines Natriumcocoylglycinats;
(ii) 0,05 bis 10 Gew.-% 2,3-Dihydroxypropyltrimethylammoniumchlorid und
(iii) Wasser.

4. Zusammensetzung nach Anspruch 3,
die ferner 0,1 bis 30 % eines Tensids aufweist.

5. Zusammensetzung nach Anspruch 4,
wobei das Tensid Natriumcocoylisethionat ist.

## Revendications

1. Système conservateur comprenant :
(I) du glycinate sodique de cocoyle ; et
(II) du chlorure de 2,3-dihydroxypropyl-triméthylammonium ; et
dans lequel le système manifeste une réduction Log contre Pseudomonas aeruginosa d'au moins 2 dans les 48 heures qui suivent son application à un système aqueux.

2. Système selon la revendication 1 ayant une réduction Log d'au moins 3,5 dans les 48 heures qui suivent son application à un système aqueux.

3. Composition d'hygiène corporelle comprenant :
(i) de 1 à 20 % en poids de glycinate sodique de cocoyle ;
(ii) de 0,05 à 10 % en poids de chlorure de 2,3-dihydroxypropyltriméthylammonium ; et
(iii) de l'eau.

4. Composition selon la revendication 3 comprenant en outre de 0,1 à 30 % de tensioactif.

5. Composition selon la revendication 4 dans laquelle le tensioactif est l'iséthionate sodique de cocoyle.
